# EUROPEAN PATENT SPECIFICATION

(11) **EP 0 715 527 B1**
(45) Date of publication and mention of the grant of the patent: **01.12.1999**
(21) Application number: 94924328.1
(22) Date of filing: 22.08.1994
(51) Int. Cl.: A61M 5/152

(54) **INFUSION APPARATUS**
INFUSIONSVORRICHTUNG
APPAREIL DE PERFUSION

(30) Priority: 20.08.1993 FI 933679
(43) Date of publication of application: 12.06.1996
(73) Proprietor: Keskiväli, Eero, SF-15900 Lahti (FI); Kyyhkynen, Heimo, SF-15320 Lahti (FI)
(72) Inventor: Keskiväli, Eero, SF-15900 Lahti (FI); Kyyhkynen, Heimo, SF-15320 Lahti (FI)
(74) Representative: Järveläinen, Pertti Tauno Juhani
(86) International application number: FI9400366
(87) International publication number: WO9505859

(56) References cited:
- DE-A- 2 246 054
- DE-A- 2 731 448

## Description

The present invention relates to an infusion apparatus for supplying an infusion liquid under pressure from an infusion liquid bag, as defined in the preamble of claim 1.

Infusion is a method of supplying a liquid into the system of a patient. The infusion liquid may be e.g. a saline solution, whole blood or plasma. The infusion liquid is contained in elastic plastic bags, from where it is passed via hoses and a cannula into the system of the patient.

The infusion liquid bag is generally suspended above the patient on a hook on a liquid bag support, so that the infusion liquid flows down into the patient's system under the influence of gravity. However, it is sometimes necessary to accelerate the liquid flow. In this case, the infusion liquid bag has to be pressurized, which means creating a pressure in the liquid so that it flows faster into the patient.

In previously known technology, this is achieved by using an arrangement in which a pressure bag made of elastic plastic is placed against the liquid bag. In a solution according to known technology, the pressure bag is pressurized by means of a hand pump.

The pressure bag is held against the infusion liquid bag e.g. by means of a net attached to the pressure bag and forming together with it a sleeve into which the infusion liquid bag can be drawn. Such a solution is presented e.g. in US patent specification 4 735 613. Attached to the upper end of the net is a loop of string which is passed through a hole in the upper end of the infusion liquid bag, enabling the bags to be suspended.

An infusion apparatus according to the first part of claim 1 is known from DE A 27 31 448 (see in particular Fig 2).

The object of the present invention is to achieve a better solution than those known in the art for supplying an infusion liquid under pressure from an infusion liquid bag. The features characteristic of the infusion apparatus of the invention are presented in the claims below.

As compared to previously known solutions, the apparatus of the invention provides the following advantages:

The apparatus of the invention enables the infusion liquid bag to be changed and pressurized considerably faster: With known techniques, this takes about 80 s., whereas with the apparatus of the invention it takes only 15 s. The situations where pressurized infusion liquid supply is needed are generally cases of emergency (e.g. operating theatres, first-aid stations, ambulances), in which it is important that the preparations for the use of a pressurized infusion liquid can be performed quickly. In critical situations (first aid/surgical operations), where the patient may be receiving several simultaneous infusions into a vein, the nurses' time is spent exclusively on changing the infusion liquid bags when previously known solutions are used.

In a known solution, the pressure has to be increased by means of a hand pump. This is more laborious than in the case of the apparatus of the invention, in which the pressure is increased by rotating a threaded part or by pulling at a handle. In large operations, tens of litres of liquid must be infused into the patient, and this is physically hard work when done with a hand pump. Moreover, in the apparatus of the invention, the threaded part can be rotated using the muscles of an arm, so this feels lighter than squeezing a hand pump.

In a known solution, when the infusion liquid bag is to be changed, the pressure bag has to be squeezed by hand to force out the air through a small valve to allow a new infusion liquid bag to be drawn into position. In addition, in a known solution, it is difficult to get the infusion liquid bag into and out of the pressure bag (the infusion liquid bags are tacky because they are preheated), whereas in the apparatus of the invention changing the infusion liquid bag (getting it in/out) is easy.

The pressure bag used in known solutions is soft and therefore difficult to handle (e.g. to suspend on a hook or to take off from one).

No electricity or compressed air is needed to operate the apparatus of the invention, so it can be used even while the bed is being moved.

In the following, the invention is described in detail by the aid of examples by referring to the attached drawings, in which
Fig. 1 presents the infusion apparatus of the invention as seen from the side of the latch.
Fig. 2 presents the infusion apparatus of the invention as seen from the side of the hinge.
Fig. 3 presents the infusion apparatus of the invention in front view.
Fig. 4 presents a situation where one half of the frame is opened and the liquid bag changed.
Fig. 5 presents the two halves of the frame together, with the latch closed.
Fig. 6 presents a situation where one half of the frame is opened and the liquid bag changed.
Fig. 7 presents another embodiment of the apparatus of the invention in top view.
Fig. 8 presents another embodiment of the apparatus of the invention in side view.

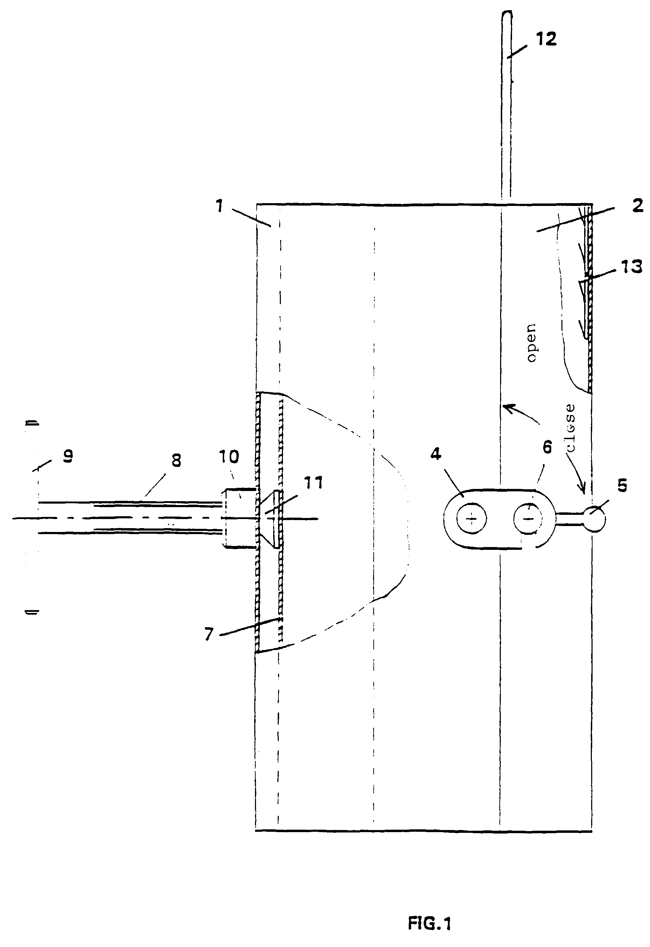

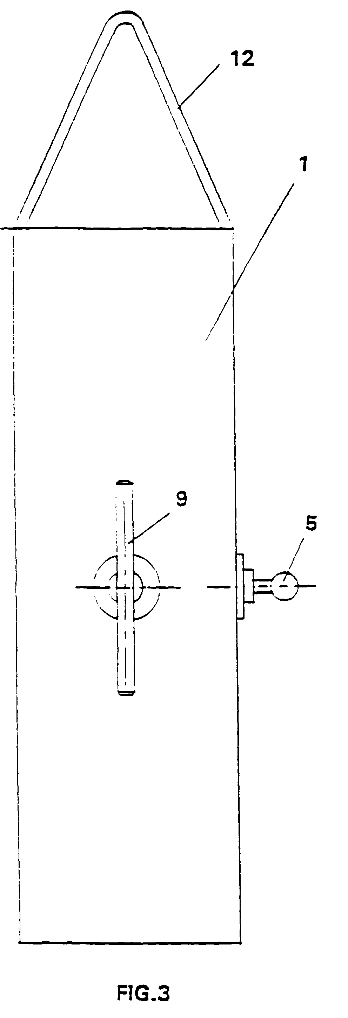

Figures 1 - 3 depict an infusion apparatus according to the invention. It has a boxlike frame which consists of two halves 1,2 and is open at the top and bottom. The first half is of a rectangular cross-section, while the other half is a trough of a semicircular shape.

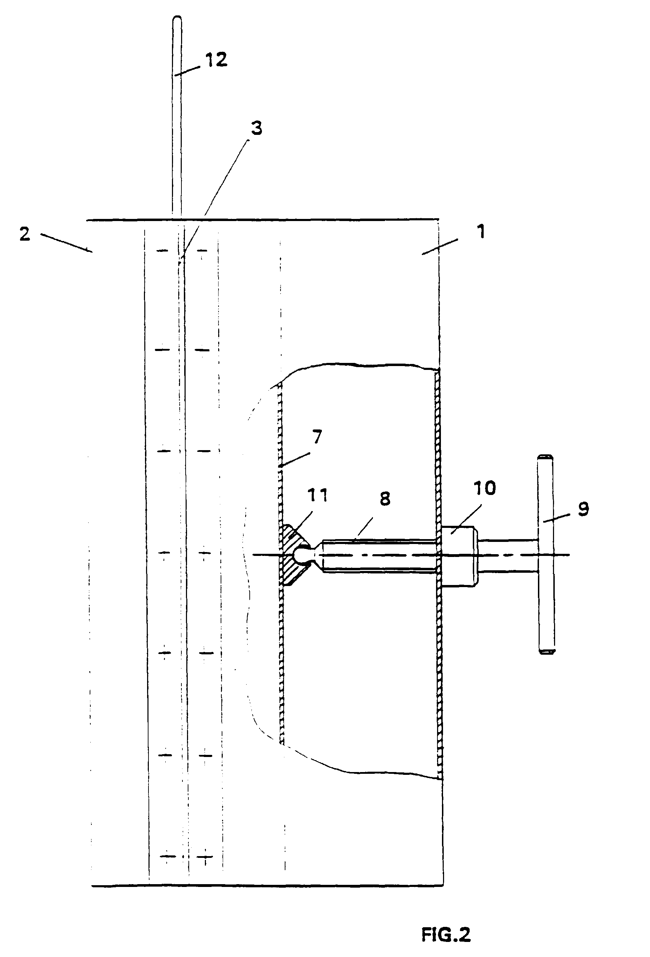

The two halves 1,2 of the frame are hinged together with a vertical hinge 3 (Fig. 2), enabling the frame to be opened and closed. When the halves are together, they can be locked to each other by means of a latch, which consists of a plate 4 pivoted on the first half 1 and provided with a lever 5 enabling it to be turned in a manner such that, when the latch is being turned to lock the halves in the closed position (Fig. 1), a notch in the plate engages a pin 6 provided on the other half 2. To open the latch, the plate 4 is turned up.

Fitted inside one half of the frame is a vertical troughlike compression flange 7 of a semicircular cross-section. The compression flange 7 can be moved inside the first half 1 of the frame in the lengthwise direction (in the direction of the long sides of the rectangle) by means of an actuator connected to it. The actuator consists of a threaded part 8, a torsion handle 9 and a lead-through sleeve 10 going through the front wall of the first frame half 1 and provided with an internal thread corresponding to the thread of the threaded part 8. The threaded part 8 is attached to the compression flange 7 via a vertically pivoted connecting part 11 which permits the compression flange 7 to turn in the lengthwise direction with respect to the connecting part but prevents turning in the lateral direction.

In addition, attached to the top edge of the frame, to the long sides of the rectangle, is a barlike hanger 12 of a shape resembling an inverted letter V to allow the apparatus to be suspended on a supporting hook above the patient.

Fig. 2 depicts the apparatus in a situation where the threaded part 8 is completely inside the first half 1 and the compression flange 7 lies at its largest distance from the front wall, whereas in Fig. 1 the threaded part 8 is completely outside the first half 1 of the frame and the compression flange 7 lies closest to the front wall. Moreover, Fig. 2 shows a suspension element 13 placed at the upper edge inside the second frame half 2 and consisting of several hooks placed one over the other, on which the infusion liquid bag can be suspended at a desired height.

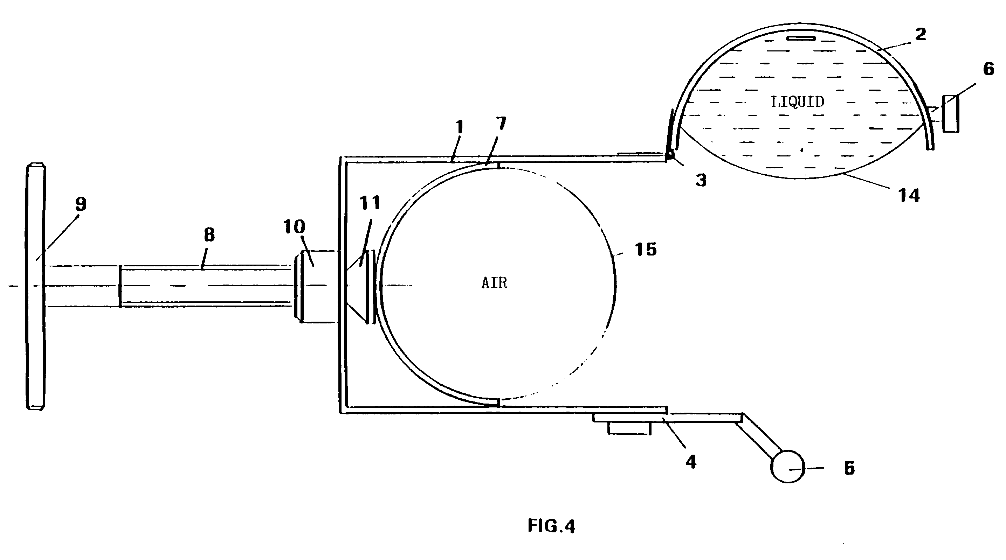

In the following, the operation of the infusion apparatus of the invention is described in greater detail by the aid of Fig. 4 - 7.

Fig. 4 illustrates a situation where the infusion liquid bag is being changed. The frame halves 1,2 have been opened, and a full infusion liquid bag 14 has been suspended in the second half 2. Fig. 4 also shows a bag 15 made of elastic plastic and filled with air. The air bag 15 is attached to the compression flange 7 on the side facing the liquid bag 14 and used between the compression flange 7 and the liquid bag as a power transmitting air buffer which distributes the pressure compressing the liquid bag 14 over its whole area. In Fig. 4, the compression flange 7 is in the position closest to the front wall of the first half 1. The air bag 14 is provided with means (not shown) for measuring the air pressure inside the bag, e.g. as described in the above-mentioned US patent specification 4 735 613.

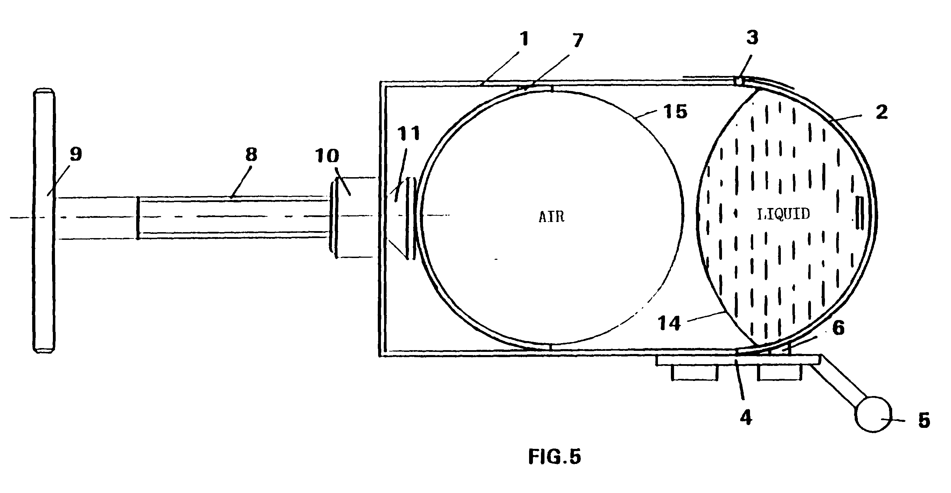

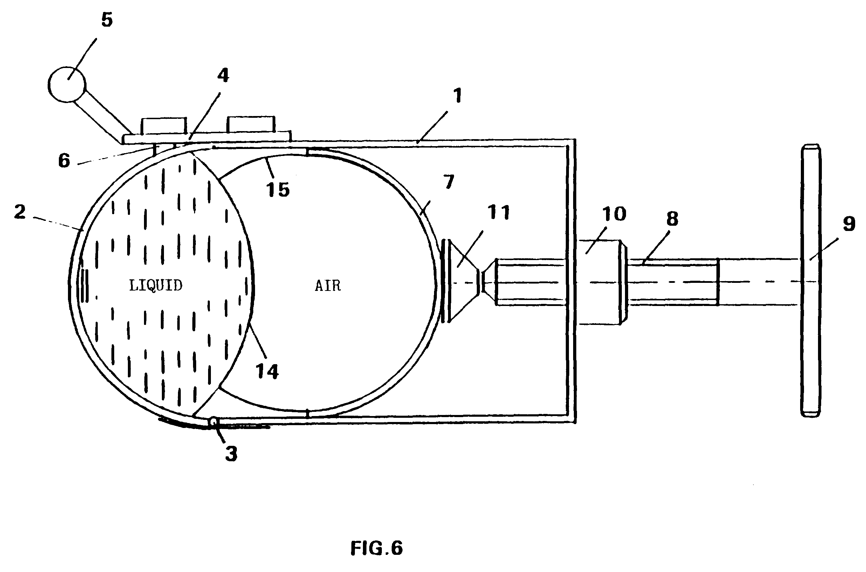

Fig. 5 presents a situation where the two halves 1,2 of the frame have been turned together and the latch has been closed. Fig. 6 shows a situation where the air buffer 15 is pressed against the infusion liquid bag 14 as the threaded part 8 is being turned by means of the torsion handle 9. In this way, the pressure of the infusion liquid is increased to a desired level, typically to a value of 150 - 300 mmHg.

The diameter of the air bag 15 is typically 100 mm. Correspondingly, the diameter of the compression flange moving inside the first half 1 of the frame as well as that of the second frame half is 100 mm. The length of the threaded part 8 is so chosen that, when the threaded part 8 is in its extreme position, the compression flange 7 and the second half 2 are pressed against each other and the tube thus formed is completely filled by the air bag. This ensures complete emptying of the liquid bag. The length of the threaded part 8 as well as the length of the long side of the frame have to be so chosen that, when the compression flange 7 is closest to the front wall of the frame, there is enough room for a full infusion liquid bag 14 inside the frame when the frame halves 1,2 are closed.

The height of the frame as well as that of the compression flange 7 correspond to the height of the air buffer and that of the liquid bags commonly used. As the liquid bags typically have a volume of 1000 ml or less and the height of the air bag used for the emptying of commercially available 1000-ml infusion liquid bags is about 330 mm, the height of the frame and the compression flange can also be 330 mm.

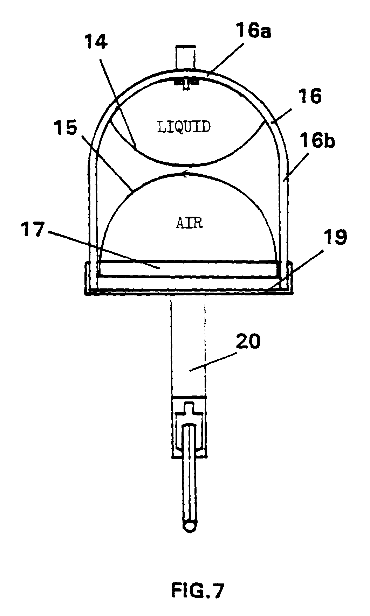

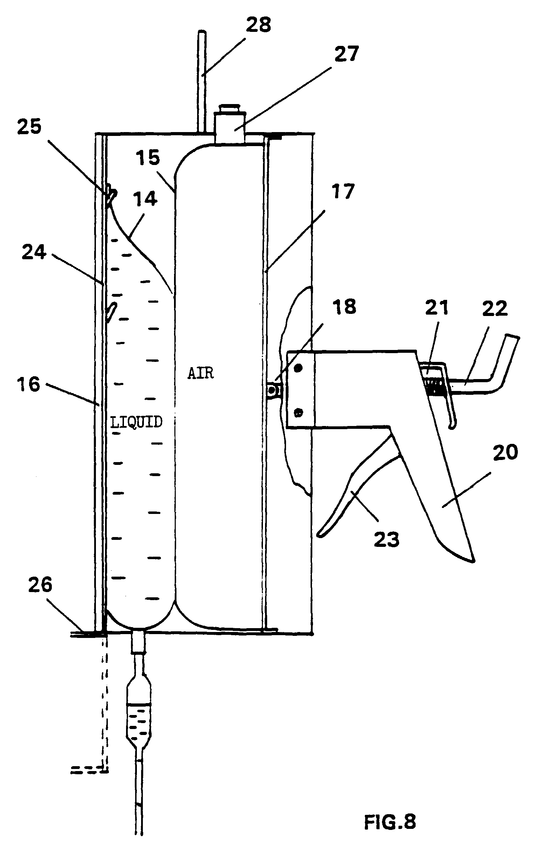

Fig. 7 and 8 present another apparatus according to the invention, It has a frame 16 consisting of one piece of plastic, e.g. polycarbonate, which has an end portion 16a of a semicircular form as seen from above and straight side portions 16b. Fitted between the sides 16b is a straight compression flange 17 which is moved by means of compression device 20 linked to it with a joint 18 and fixed to an end plate 19 attached to the ends of the side portions 16b. The compression device 20 in this example resembles a silicone gun with a spring 21, a plunger 22 and a handle 23.

The liquid bag 14 is suspended on a hook 25 in a vertical strip of plate 24 on the end wall 16a. By means of the plate 24, the bag can be pushed up from below into the frame e.g. by holding the plate by the horizontal stopper part 26 provided at its lower end. The bag is in correct position when the stopper 26 touches the bottom edge of the frame 16.

The broken line shows how the plate can be moved in the vertical direction. When the plate is in its low position, a liquid bag 14 can be placed on a hook 25 and then pushed up into the correct position, as indicated by Fig. 8. Placed between the liquid bag 14 and the compression flange 7 is an air bag 15 provided with a pressure indicator 27, as in the previous example. The frame is also provided with a hanger 28 for the suspension of the apparatus.

It is obvious to a person skilled in the art that different embodiments of the invention are not restricted to the examples described above, but that they may instead be varied within the scope of the following claims.

## Claims

1. Infusion apparatus for supplying an infusion liquid under pressure from an infusion liquid bag (14), said apparatus comprising a frame (1,2,16) inside which the infusion liquid bag (14) can be placed, a compression unit used to compress the infusion liquid bag (14) so as to generate a pressure in the infusion liquid, and a pressure gauging unit (27) for measuring the pressure of the infusion liquid,
the compression unit containing a compression flange (7,17) fitted inside the frame and, connected to the flange, an actuating mechanism (8-10,20) for the generation of a compression load, **characterized** in that the compression unit contains an intermediate element (15) placable between the compression flange (7,17) and the infusion liquid bag (14) and containing a gaseous medium, said intermediate element (15) being made of an elastic material and transmitting the compressive pressure applied to the compression flange (7,17) to the infusion liquid bag (14).

2. Apparatus according to claim 1, **characterized** in that the frame (1,2,16) is open at least at its upper or lower end.

3. Apparatus according to claim 1 or 2, **characterized** in that it has inside the frame (16) a platelike attaching part (24) movable in a vertical direction, to which is attached at least one attaching part (25) for the attachment of a liquid bag (14), and by means of which the liquid bag (14) can be placed inside the frame.

4. Infusion apparatus according to claim 1, **characterized** in that the frame consists of two halves (1,2) which are hinged together to permit the frame to be opened and closed and which can be locked to each other with a latch device (4-6) designed to lock the two halves (1,2) of the frame together.

5. Infusion apparatus according to claim 1, **characterized** in that the mechanism actuating the compression flange (7,17) consists of a slide shaft (22), a spring (21) pushing it and a handle (23) moving the shaft.

6. Infusion apparatus according to claim 1, **characterized** in that the mechanism actuating the compression flange (7,17) consists of a threaded part (8) fitted in the frame, a sleeve (10) fixed to the frame and provided with an internal thread corresponding to the thread of the threaded part (8), and an actuating means (9) for rotating the threaded part (8).

7. Infusion apparatus according to claim 6, **characterized** in that the threaded part (8) is attached to the compression flange (7,17) via a vertically pivoted connecting part (11) which permits the compression flange (7) to turn in the vertical direction with respect to the point of attachment.

8. Infusion apparatus according to claim 1, **characterized** in that the actuating mechanism is connected to the compression flange via a hinged joint (11,18).

## Patentansprüche

1. Infusionsvorrichtung zum Zuführen von Infusionsflüssigkeit unter Druck aus einem Infusionsflüssigkeitsbeutel (14), welche Vorrichtung einen Rahmen (1, 2, 16), in weichem der Infusionsflüssigkeitsbeutel (14) angeordnet werden kann, eine Kompressionseinheit, die zum Zusammendrücken des Infusionsflüssigkeitsbeutels (14) zum Erzeugen eines Druckes in der Infusionsflüssigkeit verwendet wird, und eine Druckmeßeinheit (27) zum Messen des Druckes der Infusionsflüssigkeit aufweist, wobei die Kompressionseinheit einen innerhalb des Rahmens eingepaßten Druckflansch (7, 17) und, verbunden mit dem Flansch, einen Betätigungsmechanismus (8 bis 10, 20) zum Erzeugen einer Druckladung enthält,
**dadurch gekennzeichnet,**
**daß** die Kompressionseinheit ein zwischen dem Druckflansch (7, 17) und dem Infusionsflüssigkeitsbeutel (14) plazierbares und ein gasförmiges Medium enthaltendes Zwischenelement (15) enthält, weiches Zwischenelement (15) aus einem elastischen Material hergestellt ist und den dem Druckflansch (7, 17) zugeführten Kompressionsdruck auf den Infusionsflüssigkeitsbeutel (14) überträgt.

2. Vorrichtung nach Anspruch 1,
**dadurch gekennzeichnet,**
**daß** der Rahmen (1, 2, 16) wenigstens an seinem oberen oder unteren Ende offen ist.

3. Vorrichtung nach Anspruch 1 oder 2,
**dadurch gekennzeichnet,**
**daß** sie innerhalb des Rahmens (16) ein plattenähnliches in einer vertikalen Richtung bewegliches Befestigungsteil (24) hat, an weichem wenigstens ein Befestigungsteil (25) zur Befestigung eines Flüssigkeitsbeutels (14) angebracht ist und mittels weichem der Flüssigkeitsbeutel (14) innerhalb des Rahmens plaziert werden kann.

4. Infusionsvorrichtung nach Anspruch 1,
**dadurch gekennzeichnet,**
**daß** der Rahmen aus zwei Hälften (1, 2) besteht, weiche aneinander angelenkt sind, um das Öffnen und Schließen des Rahmens zu erlauben, und weiche mittels einer Rastvorrichtung (4 bis 6) ineinander eingeklinkt werden können, die so aufgebaut ist, daß sie die beiden Hälften (1, 2) des Rahmens zusammenachileßen kann.

5. Infusionsvorrichtung nach Anspruch 1,
**dadurch gekennzeichnet,**
**daß** der den Druckflansch (7, 17) betätigende Mechanismus aus einer Gleitwelle (22), einer darauf drückenden Feder (21) und einem die Welle bewegenden Handgriff (23) besteht.

6. Infusionsvorrichtung nach Anspruch 1,
**dadurch gekennzeichnet,**
**daß** der den Kompressionsflansch (7, 17) betätigende Mechanismus aus einem in dem Rahmen eingepaßten Gewindeteil (8), einer an dem Rahmen befestigten und mit einem inneren Gewinde korrespondierend zu dem Gewinde des Gewindeteils (8) versehenen Hülse (10) und mit einer Betätigungseinrichtung (9) zum Rotieren des Gewindeteils (8) besteht.

7. Infusionsvorrichtung nach Anspruch 6,
**dadurch gekennzeichnet,**
**daß** der Gewindeteil (8) an dem Druckflansch (7, 17) mittels eines vertikal schwenkbaren Verbindungsteils (11) befestigt ist, weicher dem Druckflansch (7) erlaubt, sich in vertikaler Richtung bezüglich des Befestigungspunktes zu drehen.

8. Infusionsvorrichtung nach Anspruch 1,
**dadurch gekennzeichnet,**
**daß** der Betätigungsmechanismus an dem Druckflansch mittels einer scharnierartigen Verbindung (11, 18) verbunden ist.

## Revendications

1. Appareil de perfusion destiné à une alimentation d'un liquide de perfusion sous pression à partir d'une poche de liquide de perfusion (14), ledit appareil comprenant un bâti (1, 2, 16) à l'intérieur duquel la poche de liquide de perfusion (14) peut être placée, une unité de compression utilisée pour comprimer la poche de liquide de perfusion (14) de façon à engendrer une pression dans le liquide de perfusion, et une unité de mesure de pression (27) destinée à mesurer la pression du liquide de perfusion, l'unité de compression contenant un flasque de compression (7, 17) adapté à l'intérieur du bâti et, relié au flasque, un mécanisme d'actionnement (8 à 10, 20) destiné à la génération d'une charge de compression, caractérisé en ce que l'unité de compression contient un élément intermédiaire (15) gui peut être placé entre le flasque de compression (7, 17) et la poche de liquide de perfusion (14) et contenant un milieu gazeux, ledit élément intermédiaire (15) étant fait d'un matériau élastique et transmettant la pression de compression appliquée au flasque de compression (7, 17) à la poche de liquide de perfusion (14).

2. Appareil selon la revendication 1, caractérisé en ce que le bâti (1, 2, 16) est ouvert au moins au niveau de son extrémité supérieure ou inférieure.

3. Appareil selon la revendication 1 ou 2, caractérisé en ce qu'il comporte à l'intérieur du bâti (16) une partie de fixation en forme de plaque (24) qui peut être déplacée dans une direction verticale, à laquelle est fixée au moins une partie de fixation (25) destinée à la fixation d'une poche de liquide (14), et au moyen de laquelle la poche de liquide (14) peut être placée à l'intérieur du bâti.

4. Appareil de perfusion selon la revendication 1, caractérisé en ce que le bâti est constitué de deux moitiés (1, 2) qui sont articulées ensemble afin de permettre que le bâti soit ouvert et fermé et qul peuvent être verrouillées l'une à l'autre avec un dispositif de verrou (4 à 6) conçu pour verrouiller les deux moitiés (1, 2) du bâti ensemble.

5. Appareil de perfusion selon la revendication 1, caractérisé en ce que le mécanisme actionnant le flasque de compression (7, 17) est constitué d'un axe coulissant (22), d'un ressort (21) qui le pousse et d'une poignée (23) qui déplace l'axe.

6. Appareil de perfusion selon la revendication 1, caractérisé en ce que le mécanisme actionnant le flasque de compression (7, 17) est constitué d'une partie filetée (8) adaptée dans le bâti, d'un manchon (10) fixé au bâti et muni d'un filetage interne correspondant au filetage de la partie filetée (8), et d'un moyen d'actionnement (8) destiné à faire tourner la partie filetée (8).

7. Appareil de perfusion selon la revendication 6, caractérisé en ce que la partie filetée (8) est fixée au flasque de compression (7, 17) par l'intermédiaire d'une partie de liaison pivotant verticalement (11) qui permet que le flasque de compression (7) tourne dans la direction verticale par rapport au point de fixation.

8. Appareil de perfusion selon la revendication 1, caractérisé en ce que le mécanisme d'actionnement est relié au flasque de compression par l'intermédiaire d'un joint articulé (11, 18).
